# EUROPEAN PATENT SPECIFICATION

(11) **EP 4 212 499 B1**
(45) Date of publication and mention of the grant of the patent: **04.03.2026**
(21) Application number: 22213722.6
(22) Date of filing: 03.01.2007
(51) Int. Cl.: C07C 17/087, C07C 21/18, C07C 17/04, C07C 19/01, C07C 17/06, C07C 19/10, C07C 17/20, C07C 17/21, C07C 17/25

(54) **METHOD FOR PRODUCING FLUORINATED ORGANIC COMPOUNDS**
VERFAHREN ZUR HERSTELLUNG FLUORIERTER ORGANISCHER VERBINDUNGEN
PROCÉDÉ DE PRODUCTION DE COMPOSÉS ORGANIQUES FLUORÉS

(30) Priority: 03.01.2006 US 75548506 P
(43) Date of publication of application: 19.07.2023
(62) Divisional of application: 17201423.5
(73) Proprietor: Honeywell International Inc., Morris Plains, NJ 07950 (US)
(72) Inventor: MERKEL, Daniel, West Seneca, 14224 (US); TUNG, Hsueh Sung, Getzville, 14068 (US); VAN DER PUY, Michael, Amherst, 14221 (US); MA, Jing Ji, Morristown, 07962 (US); DUBEY, Rajesh, Buffalo, 14210 (US); LIGHT, Barbara, Niagra Falls, 14305 (US); BORTZ, Cheryl, N Tonawanda, 14120 (US); PHILLIPS, Steven D, Buffalo, 14218 (US); MUKHOPAHYAY, Sudip, Morristown, 07962 (US)
(74) Representative: Stepney, Gregory John

(56) References cited:
- HENNE ALBERT L ET AL: "Fluorinated derivatives of propane and propylene. VI", JOURNAL OF THE AMERICAN CHEMICAL SOCIETY, AMERICAN CHEMICAL SOCIETY, vol. 68, 1 January 1946 (1946-01-01), pages 496 - 497, XP002448570, ISSN: 0002-7863, DOI: 10.1021/JA01207A041
- PALETA OLDRICH ET AL: "Synthesis of perfluoroallyl chloride and some chlorofluoropropenes", BULLETIN DE LA SOCIETE CHIMIQUE DE FRANCE, SOCIETY FRANCAISE DE CHIMIE , PARIS, FRANCE, no. 6, 1 January 1986 (1986-01-01), pages 920 - 924, XP009088473, ISSN: 0037-8968
- MARIA O BURGIN ET AL: "UNIMOLECULAR REACTION KINETICS OF CF2CLCF2CH3 AND CF2CLCF2CD3: EXPERIMENTAL EVIDENCE FOR A NOVEL 1,2-FCL REARRANGEMENT PATHWAY", THE JOURNAL OF PHYSICAL CHEMISTRY A, WASHINGTON DC, US, vol. 105, 1 January 2001 (2001-01-01), pages 1615 - 1621, XP002448571, ISSN: 1089-5639, DOI: 10.1021/JP002511D

## Description

### BACKGROUND OF INVENTION

### (1) Field of Invention:

This invention relates to novel methods for preparing fluorinated organic compounds, and more particularly to methods of producing fluorinated olefins.

### (2) Description of Related Art:

Hydrofluorocarbons (HFCs), in particular hydrofluoroalkenes such tetrafluoropropenes (including 2,3,3,3-tetrafluoro-1-propene (HFO-1234yf) and 1,3,3,3-tetrafluoro-1-propene (HFO-1234ze) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkenes are known. For example, U.S. Pat. No. 4,900,874 (Ihara et al) describes a method of making fluorine containing olefins by contacting hydrogen gas with fluorinated alcohols. Although this appears to be a relatively high-yield process, for commercial scale production the handling of hydrogen gas at high temperature raises difficult safety related questions. Also, the cost of producing hydrogen gas, such as building an on-site hydrogen plant, can be in many situations prohibitive.

U.S. Pat. No. 2,931,840 (Marquis) describes a method of making fluorine containing olefins by pyrolysis of methyl chloride and tetrafluoroethylene or chlorodifluoromethane. This process is a relatively low yield process and a very large percentage of the organic starting material is converted in this process to unwanted and/or unimportant byproducts..

The preparation of HFO-1234yf from trifluoroacetylacetone and sulfur tetrafluoride has been described. *See* Banks, et al., Journal of Fluorine Chemistry, Vol. 82, Iss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

Henne Albert L et al., "Fluorinated Derivatives of Propane and Propylene. VI", Journal of the American Chemical Society, American Chemical Society, vol. 68, 1946-01-01, pages 496-497, discloses synthesis of fluorinated derivatives of propane and propylene.

Paleta Oldrich et al., "Synthesis of Perfluoroallylchloride and Some Chlorofluoropropenes", Bulletin de la Societe Chimique de France, Society Francaise de Chimie, no. 6, 1986-01-01, pages 920-924 discloses the preparation of fluorinated propenes containing chlorine atoms using several general procedures.

Marian O Burgin et al., "Unimolecular Reaction Kinetics of CF2ClCF2CH3 and CF2ClCF2CD3: Experimental Evidence for a Novel 1,2-FCl Rearrangement Pathway", The Journal of Physical Chemistry A, vol. 105, 2001-01-01, pages 1615-1621 discloses the reaction of CF₂ClCF₂CH₃ and CF₂ClCF₂CD₃ via elimination of HF (DF) and also by a 1,2-FCl rearrangement.

### SUMMARY

Applicants have discovered a method for producing fluorinated organic compounds, including hydrofluoropropenes, which comprises converting at least one compound of Formula (IAA):

CH₂=CClCF₃ (IAA)

to the compound

CF₃CF₂CH₃

and subsequently converting the CF₃CF₂CH₃ (245cb) to a compound of Formula (II) (CF₃CF=CH₂ (1234yf)).

As used herein and throughout, unless specifically indicated otherwise, the term "converting" includes directly converting (for example, in a single reaction or under essentially one set of reaction conditions, an example of which is described hereinafter) and indirectly converting (for example, through two or more reactions or using more than a single set of reaction conditions).

### DETAILED DESCRIPTION OF PREFERRED EMBODIMENTS

### FLUORINATION OF THE COMPOUND OF FORMULA IAA

The compound of Formula (IAA), is subjected to fluorination reaction(s) to produce CF₃CF₂CH₃ (245cb). Preferably this gas phase reaction is at least partially catalyzed.

### DEHYDROHALOGENATION OF CF₃CF₂CH₃ (245cb)

The compound CF₃CF₂CH₃ (245cb) is dehydrofluorinated to CF₃CF=CH₂ (1234yf).

### EXAMPLES

Additional features of the present invention are provided in the following examples.

### Example 1 (Reference Example)

### Selective catalyzed-transformation of CCl₂=CClCH₂Cl to CF₃CCl=CH₂ (HFO-1233xf) in gas-phase

A (56 cm) 22-inch long and 1.27 cm 1/2-inch diameter Monel pipe gas-phase reactor is charged with about 120 cc of a catalyst or a mixture of two catalysts. In case of a mixture, Cr₂O₃ catalyst is kept at the bottom zone of the reactor at a constant temperature of about 270°C-500°C and the other catalyst, such as FeCl₃/C, is kept at the middle and the top zone of the reactor at a constant temperature of about 120°C - 220°C. The reactor is mounted inside a heater with three zones (top, middle, and bottom). The reactor temperature is read by custom-made-5-point thermocouples kept inside at the middle of the reactor. The bottom of the reactor is connected to a preheater, which is kept at 300°C by electrical heating. The liquid-HF is fed from a cylinder into the pre-heater through a needle valve, liquid mass-flow meter, and a research control valve at a constant flow of about 1 to about 1000 grams pre hour (g/h). The HF cylinder is kept at a constant pressure of 412 kPa (45 psig) by applying anhydrous N₂ gas pressure into the cylinder head space. About 10 to about 1000 g/h of CCl₂=CClCH₂Cl is fed as a liquid through a dip tube from a cylinder under about 412 kPa (45 psig) of N₂ pressure. The organic flows from the dip tube to the preheater (kept at about 250°C) through a needle valve, liquid mass-flow meter, and a research control valve at a constant flow of 1-1000 g/h. The organic is also fed as a gas while heating the cylinder containing organic at about 220°C. The gas coming out of the cylinder is passed through a needle valve and a mass flow controller into the preheater. The organic line from the cylinder to the pre-heater is kept at about 200°C by wrapping with constant temperature heat trace and electrical heating elements. All feed cylinders are mounted on scales to monitor their weight by difference. The catalysts are dried at the reaction temperature over a period of about 8 hours and then pretreated with about 50 g/h of HF under atmospheric pressure over a period of about 6 hours and then under 446 kPa (50 psig) HF pressure over another period of about 6 hours before contacting with organic feed containing CCl₂=CClCH₂Cl. The reactions are run at a constant reactor pressure of about 101 to about 1136 kPa (about 0 to about 150 psig) by controlling the flow of reactor exit gases by another research control valve. The gases exiting reactor are analyzed by on-line GC and GC/MS connected through a hotbox valve arrangement to prevent condensation. The conversion of CCl₂=CClCH₂Cl is about 70 to about 100% and the selectivity to 1233xf is about 80% to about 95%, respectively. The product is collected by flowing the reactor exit gases through a scrubber solution comprising about 20 wt% to about 60 wt% KOH in water and then trapping the exit gases from the scrubber into a cylinder kept in dry ice or liquid N₂. The product, 1233xf is then substantially isolated by distillation. The results are tabulated in Table 1.

**Table 1: Transformation of CCl₂=CClCH₂Cl to CF₃CCl=CH₂ (CCl₂=CClCH₂Cl + 3HF → CF₃CCl=CH₂ + 3HCl)**

| **#** | **Catalyst** | **T, ^{O}C** | **HF flow, g/h** | **CCl₂=CClCH₂Cl flow, g/h** | **% Conv of CCl₂=CClCH₂Cl** | **% Sel to 1233xf** |
|---|---|---|---|---|---|---|
| **1** | 10% v/v Cr₂O₃-90% v/v FeCl₃/C | 350/150 | 50 | 12 | 79 | 81 |
| **2** | 20% v/v Cr₂O₃-80% v/v FeCl₃/C | 350/150 | 50 | 12 | 83 | 86 |
| **3** | 30% v/v Cr₂O₃-70% v/v FeCl₃/C | 350/150 | 50 | 12 | 89 | 96 |
| **4** | 30% v/v Cr₂O₃-70% v/v FeCl₃/C | 350/150 | 70 | 12 | 79 | 93 |
| **5** | 30% v/v Cr₂O₃-70% v/v FeCl₃/C | 345/170 | 50 | 25 | 85 | 90 |
| **6** | Cr₂O₃ | 350 | 50 | 20 | 90 | 93 |
| **7** | FeCl₃/C | 150 | 50 | 20 | 74 | 39 |
| **8** | SbCl₅/C | 150 | 50 | 20 | 81 | 52 |

| | | | | | | |
|---|---|---|---|---|---|---|
| Reaction conditions: Catalyst used (total) 120 cc; pressure, 122 kPa (1.5 psig); | | | | | | |

### Examples 2A and 2B

### Liquid-phase catalytic fluorination of CF₃CCl=CH₂ (1233xf) with HF to CF₃CFClCH₃ (244bb)

### Example 2A

About 327 grams of HF, about 50 grams 1233xf, and about 75 grams SbCl₅ were charged into a 1-L autoclave. The reaction mixture was stirred at a temperature of about 80°C for about 3 hours under about 4376 kPa (620 psig) of pressure. After the reaction, the reactor was cooled to about 0°C and about 300 ml water was then added slowly into the autoclave over a period of about 45 min. After complete addition of water under stirring, the reactor was cooled to room temperature and then the overhead gases were transferred to another collecting cylinder. The yield of CF₃CFClCH₃ was about 90% at a 1233xf conversion level of about 98%. The other major by-products were CF₃CF₂CH₃ (2%), and an unidentified isomer of a C4 compound of the general formula, C₄H₃Cl₃F₄ (8%).

### Example 2B

About 327 grams HF, about 50 grams 1233xf, and about 75 grams SbCl₅ were charged into a 1-L autoclave. The reaction mixture was stirred at 80°C for about 3 hours under about 4411 kPa (625 psig) of pressure. After the reaction, the reactor was cooled to about 45°C and then the overhead gas mixture was passed through a well dried KF, NaF, or Al₂O₃ (350 g) packed column kept at about 80°C to strip off HF from the gas stream. The gases coming out of the column are collected in a cylinder kept in dry ice (-70^{O}C) bath. The yield of CF₃CFClCH₃ was 87% at a 1233xf conversion level of 93%. The other major by-products were CF₃CF₂CH₃ (1%), and an unidentified isomer of a C4 compound of the general formula, C₄H₃Cl₃F₄ (7%). The product, CF₃CFClCH₃ was isolated by distillation with 98% purity.

### Example 3 (Reference Example)

### Selective catalyzed-transformation of CCl₃CCl=CH₂ to CF₃CCl=CH₂ (HFO-1233xf) in gas-phase

A 56 cm (22-inch) long and 1.27 cm (1/2-inch) diameter Monel pipe gas phase reactor was charged with 120 cc of a catalyst or a mixture of two catalysts. In case of a mixture, Cr₂O₃ catalyst is kept at the bottom zone of the reactor at a substantially constant temperature of from about 270°C to about 500°C and the other catalyst, such as FeCl₃/C is kept at the middle and the top zone of the reactor at a substantially constant temperature of from about 120°C to about 220°C. The reactor was mounted inside a heater with three zones (top, middle, and bottom). The reactor temperature was read by custom-made-5-point thermocouples kept inside at the middle of the reactor. The bottom of the reactor was connected to a pre-heater, which was kept at about 300°C by electrical heating. The liquid-HF was fed from a cylinder into the pre-heater through a needle valve, liquid mass-flow meter, and a research control valve at a substantially constant flow of from about 1 to about 1000 g/h. The HF cylinder was kept at a substantially constant pressure of about 412 kPa (45 psig) by applying anhydrous N₂ gas pressure into the cylinder head space. A feed rate of from about 10 g/h to about 1000 g/h of CCl₃CCl=CH₂ was fed as a liquid through a dip tube from a cylinder under about (412 kPa (45 psig) of N₂ pressure. The organic was flown from the dip tube to the pre-heater (kept at about 250°C) through needle valve, liquid mass-flow meter, and a research control valve at a substantially constant flow of from about 1 to about 1000 g/h. The organic is also fed as a gas while heating the cylinder containing organic at about 220°C. The gas effluent from the cylinder is passed through a needle valve and a mass flow controller into the pre-heater. The organic line from the cylinder to the pre-heater was kept at about 200°C by wrapping with constant temperature heat trace and electrical heating elements. All feed cylinders were mounted on scales to monitor their weight by difference. The catalysts were dried at the reaction temperature over a period of about 8 hours and then pretreated with about 50 g/h of HF under atmospheric pressure over a 6 hour period and then under about 446 kPa (50 psig) HF pressure over a 6 hour period before contacting with organic feed, CCl₃CCl=CH₂. The reactions were run at a substantially constant reactor gauge pressure ranging from about 101 to about 791 kPa (about 0 to about 150 psig) by controlling the flow of reactor exit gases by another research control valve. Those gases exiting reactor were analyzed by on-line GC and GC/MS connected through a hotbox valve arrangements to prevent condensation. The conversion of CCl₃CCl=CH₂ was in a range of from about 90% to about 100% and the selectivity to CF₃CCl=CH₂ (1233xf) was about 79%. The effluent contained in addition HFO-1243zf in an amount of about 7.7%, 1232-isomer in an amount of about 1.3%, and 1223 in an amount of about 0.8%, and an unidentified byproduct. The product was collected by flowing the reactor exit gases through a 20-60 wt% aq. KOH scrubber solution and then trapping the exit gases from the scrubber into a cylinder kept in dry ice or liquid N₂. The product, 1233xf was then substantially isolated by distillation. Using only Cr₂O₃ catalyst, a selectivity of about 68% to 1233xf at a conversion level of about 79% was achieved.

### Examples 4A - 4D

### Direct Liquid-phase catalytic fluorination of CCl₃CCl=CH₂ with HF to

### CF₃CFClCH₃ (244-isomer)

### Example 4A

About 327 grams HF, about 50 grams CCl₃CCl=CH₂, and about 75 grams SbCl₅ were charged into a 1-L autoclave. The reaction mixture was stirred at about 80°C for about 3 hours under about (4307 kPa) 610 psig of pressure. After the reaction, the reactor was cooled to about 40°C and about 300 ml water was then added slowly into the autoclave over a period of about 45 min. After complete addition of water under stirring, the reactor was cooled to about room temperature and then the overhead gases were transferred to another collecting cylinder. The yield of CF₃CFClCH₃ was about 89% at a CCl₃CCl=CH₂ conversion level of about 88%. The other major byproducts were CF₃CF₂CH₃ (2%), and an unidentified isomer of a C4 compound of the general formula, C₄H₃Cl₃F₄ (8%).

### Example 4B

About 327 grams HF, about 50 grams CCl₃CCl=CH₂, and about 75 grams SbCl₅ were charged into a 1-L autoclave. The reaction mixture was stirred at about 100°C for about 3 hours under about (4824 kPa) 685 psig of pressure. After the reaction, the reactor was cooled to about 40°C and about 300 ml water was then added slowly into the autoclave over a period of about 45 minutes. After complete addition of water under stirring, the reactor was cooled to room temperature and then the overhead gases were transferred to another collecting cylinder. The yield of CF₃CFClCH₃ was about 78% at a CCl₃CCl=CH₂ conversion level of about 100%. The other major byproducts were CF₃CF₂CH₃ (about 4%), and an unidentified isomer of a C4 compound of the general formula, C₄H₃Cl₃F₄ (about 13%).

### Example 4C

About 327 grams HF, about 50 grams CCl₃CCl=CH₂, and about 75 grams SbCl5 were charged into a 1-L autoclave. The reaction mixture was stirred at about 125°C for about 6 hours under about (5790 kPa) 825 psig of pressure. After the reaction, the reactor was cooled to about 40°C and about 300 ml water was then added slowly into the autoclave over a period of about 45 min. After complete addition of water under stirring, the reactor was cooled to about room temperature and then the overhead gases were transferred to another collecting cylinder. The major products were CF₃CF₂CH₃ (about 53%) and CF₃CFClCH₃ (about 25%) at a CCl₃CCl=CH₂ conversion level of about 100%. The other major by-products were and unidentified isomer of a C4 compound of the general formula, C₄H₃Cl₃F₄ (8%) and tar.

### Example 4D

About 327 grams HF, about 50 grams CCl₃CCl=CH₂, and about 75 g SbCl₅ were charged into a 1-L autoclave. The reaction mixture was stirred at about 150°C for about 6 hours under about (5790 kPa) 825 psig of pressure. After the reaction, the reactor was cooled to about 40°C and about 300 ml water was then added slowly into the autoclave over a period of about 45 minutes. After complete addition of water under stirring, the reactor was cooled to about room temperature and then the overhead gases were transferred to another collecting cylinder. The major products were CF₃CF₂CH₃ (about 57%) and CF₃CFClCH₃ (about 15%) at a CCl₃CCl=CH₂ conversion level of about 100%. The other major by-products were and unidentified isomer of a C4 compound of the general formula, C₄H₃Cl₃F₄ (about 11%) and tar.

### Example 5

### Catalytic conversion of CF₃CF₂CH₃ to CF₃CF=CH₂

A (56 cm) 22-inch (1.27 cm (1/2-inch) diameter) Monel tube gas phase reactor was charged with 120 cc of a catalyst. The reactor was mounted inside a heater with three zones (top, middle and bottom). The reactor temperature was read by custom made 5-point thermocouples kept at the middle inside of the reactor. The inlet of the reactor was connected to a pre-heater, which was kept at about 300°C by electrical heating. Organic material (245cb) was fed from a cylinder kept at about 65°C through a regulator, needle valve, and a gas mass-flow-meter. The organic line to the pre-heater was heat traced and kept at a substantially constant temperature in a range of from about 65°C to about 70°C by electrical heating to avoid condensation. The feed cylinder was mounted on a scale to monitor its weight by difference. The reactions were run at a substantially constant reactor pressure of from about 101 to about 1136 kPa (about 0 to about 100 psig) by controlling the flow of reactor exit gases by another research control valve. The gas mixtures exiting reactor was analyzed by on-line GC and GC/MS connected through a hotbox valve arrangements to prevent condensation. The conversion of 245cb was in the range of from about 30% to about 70% and the selectivity to 1234yf was in the range of from about 90% 5o about 100% depending on the reaction conditions. The products were collected by flowing the reactor exit gases through a 20-60-wt% of aq. KOH scrubber solution and then trapping the exit gases from the scrubber into a cylinder kept in dry ice or liquid N₂. The products were then substantially isolated by distillation. Results are tabulated in Table 4.

**Table 4: Transformation of CF₃CF₂CH₃ to 1234yf**

| # | Cat | T, °C | H₂, sccm | CF₃CF₂CH₃ (245cb) sccm | Conversion of 245cb, % | 1234yf (Sel. %) |
|---|---|---|---|---|---|---|
| 1 | A | 575 | 0 | 65 | 79 | 63 |
| 2 | B | 575 | 0 | 68 | 82 | 57 |
| 3 | C | 575 | 0 | 73 | 73 | 61 |
| 4 | D | 575 | 0 | 68 | 84 | 59 |
| 5 | D | 575 | 20 | 68 | 89 | 73 |
| 6 | E | 550 | 0 | 69 | 92 | 53 |
| 7 | F | 550 | 0 | 67 | 93 | 33 |
| 8 | G | 550 | 0 | 69 | 73 | 46 |

**Reaction conditions:** pressure, 119-138 kPa (2.5-5.3 psig); catalyst, 100 cc, A is NORIT RFC 3; B is Shiro-Saga activated carbon; C is Aldrich activated carbon; D is Calgon activated carbon; E is 0.5 wt% Pd/C; F is 0.5 wt% Pt/C; G is Ni-mesh; Organic cylinder temperature is about 65^{O}C: CF₃CF₂CH₃ (245cb) line to the preheater is maintained at about 50^{O}C; preheater temperature is maintained at about 350°C; N₂ flow is not used; pressure is maintained at about 122 kPa (3 psig).

## Claims

1. A method for producing CF₃CF=CH₂ (1234yf), the method comprising converting CF₃CClCH₂ (1233xf) to CF₃CF₂CH₃ (245cb) and subsequently converting the CF₃CF₂CH₃ (245cb) to CF₃CF=CH₂ (1234yf).

2. The method of claim 1, wherein converting the CF₃CF₂CH₃ (245cb) to CF₃CF=CH₂ (1234yf) comprises contacting CF₃CF₂CH₃ (245cb) with a catalyst in a reactor, optionally wherein the catalyst is selected from activated carbon, Pd/C, Pt/C or Ni-mesh.

3. The method of claim 2, wherein the catalyst is selected from activated carbon, 0.5 wt% Pd/C, 0.5 wt% Pt/C or Ni-mesh.

4. The method of any preceding claim, wherein converting the CF₃CF₂CH₃ (245cb) to CF₃CF=CH₂ (1234yf) comprises converting the CF₃CF₂CH₃ (245cb) in a reactor at a temperature of 550°C to 575°C.

5. The method of any preceding claim, wherein converting the CF₃CF₂CH₃ (245cb) to CF₃CF=CH₂ (1234yf) comprises converting the CF₃CF₂CH₃ (245cb) in a reactor at a gauge pressure of up to 5.3 psi (37 kPa).

6. The method of claim 5, wherein converting the CF₃CF₂CH₃ (245cb) to CF₃CF=CH₂ (1234yf) comprises converting the CF₃CF₂CH₃ (245cb) in a reactor at a gauge pressure of from 2.5 to 5.3 psi (17 to 37 kPa).

7. The method of any preceding claim, wherein converting CF₃CClCH₂ (1233xf) to CF₃CF₂CH₃ (245cb) comprises reacting CF₃CClCH₂ with HF in the presence of a catalyst, optionally in the gas phase.

8. The method of claim 7, wherein converting CF₃CClCH₂ (1233xf) to CF₃CF₂CH₃ (245cb) is carried out batch wise, continuous, or a combination of these.

## Patentansprüche

1. Verfahren zur Herstellung von CF₃CF=CH₂ (1234yf), wobei das Verfahren die Umwandlung von CF₃CClCH₂ (1233xf) in CF₃CF₂CH₃ (245cb) und anschließend die Umwandlung von CF₃CF₂CH₃ (245cb) in CF₃CF=CH₂ (1234yf) umfasst.

2. Verfahren nach Anspruch 1, wobei die Umwandlung von CF₃CF₂CH₃ (245cb) in CF₃CF=CH₂ (1234yf) das Kontaktieren von CF₃CF₂CH₃ (245cb) mit einem Katalysator in einem Reaktor umfasst, wobei der Katalysator optional ausgewählt ist aus Aktivkohle, Pd/C, Pt/C oder Ni-Gitter.

3. Verfahren nach Anspruch 2, wobei der Katalysator ausgewählt ist aus Aktivkohle, 0,5 Gew.-% Pd/C, 0,5 Gew.-% Pt/C oder Ni-Gitter.

4. Verfahren nach einem der vorhergehenden Ansprüche, wobei die Umwandlung von CF₃CF₂CH₃ (245cb) zu CF₃CF=CH₂ (1234yf) die Umwandlung des CF₃CF₂CH₃ (245cb) in einem Reaktor bei einer Temperatur von 550 °C bis 575 °C umfasst.

5. Verfahren nach einem der vorhergehenden Ansprüche, wobei die Umwandlung von CF₃CF₂CH₃ (245cb) in CF₃CF=CH₂ (1234yf) die Umwandlung von CF₃CF₂CH₃ (245cb) in einem Reaktor bei einem Manometerdruck von bis zu 5,3 psi (37 kPa) umfasst.

6. Verfahren nach Anspruch 5, wobei die Umwandlung von CF₃CF₂CH₃ (245cb) in CF₃CF=CH₂ (1234yf) die Umwandlung von CF₃CF₂CH₃ (245cb) in einem Reaktor bei einem Manometerdruck von 2,5 bis 5,3 psi (17 bis 37 kPa) umfasst.

7. Verfahren nach einem der vorhergehenden Ansprüche, wobei die Umwandlung von CF₃CClCH₂ (1233xf) in CF₃CF₂CH₃ (245cb) das Umsetzen von CF₃CClCH₂ mit HF in Gegenwart eines Katalysators, optional in der Gasphase, umfasst.

8. Verfahren nach Anspruch 7, wobei die Umwandlung von CF₃CClCH₂ (1233xf) in CF₃CF₂CH₃ (245cb) chargenweise, kontinuierlich oder einer Kombination davon ausgeführt wird.

## Revendications

1. Procédé de production de CF₃CF=CH₂ (1234yf), le procédé comprenant la conversion du CF₃CClCH₂ (1233xf) en CF₃CF₂CH₃ (245cb) et par la suite la conversion de CF₃CF₂CH₃ (245cb) en CF₃CF=CH₂ (1234yf).

2. Procédé selon la revendication 1, dans lequel la conversion du CF₃CF₂CH₃ (245cb) en CF₃CF=CH₂ (1234yf) comprend la mise en contact du CF₃CF₂CH₃ (245cb) avec un catalyseur dans un réacteur, facultativement le catalyseur étant choisi parmi le charbon actif, Pd/C, Pt/C ou Ni-mesh.

3. Procédé selon la revendication 2, dans lequel le catalyseur est choisi parmi le charbon actif, 0,5 % en poids de Pd/C, 0,5 % en poids de Pt/C ou Ni-mesh.

4. Procédé selon une quelconque revendication précédente, dans lequel la conversion du CF₃CF₂CH₃ (245cb) en CF₃CF=CH₂ (1234yf) comprend la conversion du CF₃CF₂CH₃ (245cb) dans un réacteur à une température de 550 °C à 575 °C.

5. Procédé selon une quelconque revendication précédente, dans lequel la conversion du CF₃CF₂CH₃ (245cb) en CF₃CF=CH₂ (1234yf) comprend la conversion du CF₃CF₂CH₃ (245cb) dans un réacteur à une pression manométrique allant jusqu'à 5,3 psi (37 kPa).

6. Procédé selon la revendication 5, dans lequel la conversion du CF₃CF₂CH₃ (245cb) en CF₃CF=CH₂ (1234yf) comprend la conversion du CF₃CF₂CH₃ (245cb) dans un réacteur à une pression manométrique comprise entre 2,5 et 5,3 psi (17 et 37 kPa).

7. Procédé selon une quelconque revendication précédente, dans lequel la conversion du CF₃CClCH₂ (1233xf) en CF₃CF₂CH₃ (245cb) comprend la mise en réaction du CF₃CClCH₂ avec HF en présence d'un catalyseur, éventuellement en phase gazeuse.

8. Procédé selon la revendication 7, dans lequel la conversion du CF₃CClCH₂ (1233xf) en CF₃CF₂CH₃ (245cb) est réalisé par lot, en continu, ou une combinaison de ceux-ci.
